(19) Europäisches Patentamt
European Patent Office
Office européen des brevets

(11) Veröffentlichungsnummer: **0 174 912**
**A2**

(12) # EUROPÄISCHE PATENTANMELDUNG

(21) Anmeldenummer: **85810410.2**

(22) Anmeldetag: **09.09.85**

(51) Int. Cl.⁴: **A 61 K 31/66**
C 07 F 9/09

(30) Priorität: **13.09.84 CH 4378/84**

(43) Veröffentlichungstag der Anmeldung:
**19.03.86 Patentblatt 86/12**

(84) Benannte Vertragsstaaten:
**BE CH DE FR GB IT LI LU NL SE**

(71) Anmelder: **CIBA-GEIGY AG**
**Klybeckstrasse 141**
**CH-4002 Basel(CH)**

(72) Erfinder: **Baschang, Gerhard, Dr.**
**Bückenweg 7**
**CH-4126 Bettingen(CH)**

(72) Erfinder: **Hartmann, Albert, Dr.**
**Steingasse 21A**
**D-7889 Grenzach(DE)**

(72) Erfinder: **Hirt, Hans, Dr.**
**Jupiterstrasse 20**
**CH-4123 Allschwil(CH)**

(72) Erfinder: **Lukas, Bohumir, Dr.**
**Rheinfelderstrasse 21**
**CH-4058 Basel(CH)**

(72) Erfinder: **Wirz, Peter, Dr.**
**Entenweidstrasse 18**
**CH-4142 Münchenstein(CH)**

(54) **Phosphorverbindungen, Verfahren zu ihrer Herstellung und ihre Verwendung.**

(57) Kephalinderivate der Formel I

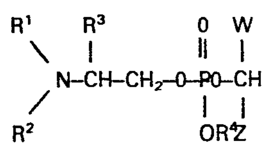

(I)

worin $R^1$ und $R^2$ unabhängig voneinander Wasserstoff oder Niederalkyl, $R^3$ Wasserstoff, Niederalkoxycarbonyl, Carbamoyl oder freies oder geschütztes Carboxy, $R^4$ Wasserstoff oder einen aliphatischen, aromatischen, aromatisch-aliphatischen oder cycloaliphatischen Rest, W Wasserstoff und Z eine 1,2-Dihydroxy-ethyl-, 2-Hydroxy-ethyl-, oder Hydroxymethyl-Gruppe, in der mindestens eine Hydroxygruppe mit einer aliphatischen $C_{6-30}$-Carbonsäure verestert oder mit einem aliphatischen $C_{6-30}$-Alkohol verethert ist, und in der die andere Hydroxygruppe, falls vorhanden, frei, mit einer aliphatischen $C_{2-30}$-Carbonsäure verestert oder mit einem aliphatischen $C_{1-30}$- Alkohol verethert ist, oder W Hydroxymethyl oder eine Hydroxymethylgruppe, die mit einer aliphatischen $C_{6-30}$-Carbonsäure verestert oder mit einem aliphatischen $C_{6-30}$-Alkohol verethert ist und Z eine Hydroxymethylgruppe, die mit einer aliphatischen $C_{6-30}$-Carbonsäure verestert oder mit einem aliphatischen $C_{6-30}$-Alkohol verethert ist, bedeuten, und deren Salze eignen sich zur Prophylaxe une Therapie von Virusinfektionen bei Warmblütern.

EP 0 174 912 A2

Croydon Printing Company Ltd.

CIBA-GEIGY AG
Basel (Schweiz)

4-15070/+

# Phosphorverbindungen, Verfahren zu ihrer Herstellung und ihre Verwendung

Die Erfindung betrifft die Verwendung von Kephalinderivaten der Formel I,

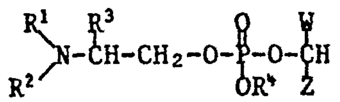

(I)

worin $R^1$ und $R^2$ unabhängig voneinander Wasserstoff oder Niederalkyl, $R^3$ Wasserstoff, Niederalkoxycarbonyl, Carbamoyl oder freies oder geschütztes Carboxy, $R^4$ Wasserstoff oder einen aliphatischen, aromatischen, aromatisch-aliphatischen oder cycloaliphatischen Rest, W Wasserstoff und Z eine 1,2-Dihydroxy-ethyl-, 2-Hydroxy-ethyl-, oder Hydroxymethyl-Gruppe, in der mindestens eine Hydroxygruppe mit einer aliphatischen $C_{6-30}$-Carbonsäure verestert oder mit einem aliphatischen $C_{6-30}$-Alkohol verethert ist, und in der die andere Hydroxygruppe, falls vorhanden, frei, mit einer aliphatischen $C_{2-30}$-Carbonsäure verestert oder mit einem aliphatischen $C_{1-30}$-Alkohol verethert ist, oder W Hydroxymethyl oder eine Hydroxymethylgruppe, die mit einer aliphatischen $C_{6-30}$-Carbonsäure verestert oder mit einem aliphatischen $C_{6-30}$-Alkohol verethert ist und Z eine Hydroxymethylgruppe, die mit einer aliphatischen $C_{6-30}$-Carbonsäure verestert oder mit einem aliphatischen $C_{6-30}$-Alkohol verethert ist, bedeuten, und von Salzen dieser Verbindungen zur Prophylaxe und Therapie von Virusinfektionen bei Warmblütern einschliesslich des Menschen sowie zur Herstellung von pharmazeutischen Präparaten für die Anwendung zur Prophylaxe und Therapie von Virusinfektionen bei

Warmblütern einschliesslich des Menschen oder als Zwischenprodukte zur Herstellung von Verbindungen der Formel I, die zur Prophylaxe und Therapie von Virusinfektionen geeignet sind.

Die Erfindung betrifft auch eine Methode zur Prophylaxe und Therapie von Virusinfektionen bei Warmblütern einschliesslich des Menschen, dadurch gekennzeichnet, dass man eine antiviral wirksame Menge einer Verbindung der Formel I oder eines pharmazeutisch verwendbaren Salzes davon verabreicht.

Die Erfindung betrifft weiter neue pharmazeutische Präparate, die als Wirkstoff, insbesondere als alleinigen Wirkstoff, eine solche pharmazeutisch verwendbare Verbindung der Formel I oder ein pharmazeutisch verwendbares Salz einer solchen Verbindung zusammen mit einem pharmazeutischen Trägermaterial enthalten.

Die Erfindung betrifft auch die Verbindungen der Formel I aus der obengenannten Gruppe, deren Anwendung in einem Verfahren zur therapeutischen Behandlung des menschlichen oder tierischen Körpers nicht zum Stand der Technik gehört, und pharmazeutisch verwendbare Salze dieser Verbindungen zur Anwendung in einem Verfahren zur therapeutischen Behandlung des menschlichen oder tierischen Körpers, pharmazeutische Präparate, enthaltend diese Verbindungen und Verfahren zu ihrer Herstellung.

Die Erfindung betrifft ferner die neuen Verbindungen der Formel I und Salze dieser Verbindungen, pharmazeutische Präparate, enthaltend eine solche Verbindung oder ein solches Salz als Wirkstoff, deren Verwendung als Arzneimittel sowie Verfahren zur Herstellung dieser Verbindungen und ihrer Salze.

Niederalkyl $R^1$ und $R^2$ ist insbesondere Methyl oder Ethyl.

Niederalkoxycarbonyl $R^3$ ist insbesondere Methoxycarbonyl oder Ethoxycarbonyl.

Geschütztes Carboxy $R^3$ ist durch eine Carboxylschutzgruppe geschütztes Carboxy.

Schutzgruppen, ihre Einführung und Abspaltung sind beispielsweise beschrieben in "Protective Groups in Organic Chemistry", Plenum Press, London, New York 1973,und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitrobenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl,

z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Die oben und im folgenden erwähnten organischen Silylreste enthalten vorzugsweise Niederalkyl, insbesondere Methyl, als Substituenten der Silizium-Atome. Entsprechende Silylgruppen sind in erster Linie Triniederalkylsilyl, insbesondere Trimethylsilyl, ferner Dimethyl-tert.-butyl-silyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl.

Ein aliphatischer, aromatischer, aromatisch-aliphatischer oder cycloaliphatischer Rest $R^4$ ist insbesondere eine Phosphorsäureschutzgruppe, z.B. Niederalkyl, wie insbesondere Methyl, gegebenenfalls substituiertes Phenyl, z.B. 2-Chlor-phenyl oder im Phenylteil gegebenenfalls substituiertes Phenylniederalkyl, wie insbesondere Benzyl oder 4-Nitro-benzyl.

Ein cycloaliphatischer Rest $R^4$ ist insbesondere auch unsubstituiertes oder z.B. durch Niederalkyl, Hydroxy oder Niederalkanoyloxy, wie Acetoxy, substituiertes Cycloalkyl mit 5 oder 6 C-Atomen, z.B. 2,3,4,5,6-Pentahydroxy-cyclohexyl, wie 1-Desoxy-myo-inosityl, oder 2,3,4,5,6-Pentaacetoxy-cyclohexyl.

Eine aliphatische $C_{6-30}$-Carbonsäure hat vorzugsweise eine gerade Anzahl von C-Atomen, in erster Linie 12-24, vor allem 16-24, hauptsächlich 16-22, C-Atome, ist verzweigt oder vorzugsweise geradkettig und ist insbesondere eine substituierte oder vorzugsweise unsubstituierte Alkan- oder Alkensäure, wobei letztere 1-3 isolierte Doppelbindungen enthalten kann, z.B. eine in der Natur vorkommende Fettsäure, wie insbesondere Palmitin-, Oel- oder Stearinsäure, oder auch Linol- oder Linolensäure. Daneben seien als Beispiele für eine aliphatische $C_{6-30}$-Carbonsäure Caprin-, Laurin-, Nervon- oder Lignocerinsäure genannt.

Ein aliphatischer $C_{6-30}$-Alkohol hat vorzusweise eine gerade Anzahl von C-Atomen, in erster Linie 12-24, vor allem 16-24, hauptsächlich 16-22, C-Atome, ist verzweigt oder vorzugsweise geradkettig und ist insbesondere ein substituiertes oder unsubstituiertes Alkanol oder Alkenol, wobei als Substituenten z.B. Hydroxy, Amino, Alkanoylamino oder Alkenoylamino genannt seien.

Die bei der Definition des Restes Z genannte aliphatische $C_{2-30}$-Carbonsäure ist insbesondere eine gegebenenfalls substituierte Alken- oder vorzugsweise Alkansäure, z.B. Essigsäure, Propionsäure oder Buttersäure oder eine der obengenannten aliphatischen $C_{6-30}$-Carbonsäuren.

Der bei der Definition des Restes Z genannte aliphatische $C_{1-30}$-Alkohol ist insbesondere ein substituiertes oder vorzugsweise unsubstituiertes Alken- oder vorzugsweise Alkanol mit bis zu 30 C-Atomen, z.B. Methanol, Ethanol oder einer der obengenannten aliphatischen $C_{6-30}$-Alkohole.

Die vor- und nachstehend verwendeten Allgemeinbegriffe haben vorzugsweise folgende Bedeutungen:

Das Präfix "Nieder" bezeichnet Reste bis und mit 7, insbesondere bis und mit 4, Kohlenstoffatomen.

Substituenten von Phenyl sind insbesondere Niederalkyl, Halogen, Niederalkoxy, Nitro oder Niederalkoxycarbonyl.

Niederalkyl ist z.B. n-Propyl, n-Butyl, Isobutyl, sek.Butyl, tert.Butyl, ferner n-Pentyl, n-Hexyl oder n-Heptyl, in erster Linie aber Methyl, Ethyl oder Isopropyl.

Halogen ist insbesondere Chlor oder Brom, ferner Fluor oder Iod.

Niederalkoxy ist insbesondere Methoxy oder Ethoxy.

Niederalkoxycarbonyl ist insbesondere Methoxycarbonyl oder Ethoxycarbonyl.

Die Verbindungen der Formel I, worin $R^4$ Wasserstoff oder $R^3$ Carboxy bedeutet, können bei geeignetem pH-Wert als innere Salze, d.h. in zwitterionischer Form, vorliegen.

Die Verbindungen der Formel I können, z.B. wenn sie mehr als eine basische Gruppe enthalten, auch Säureadditionssalze mit externen Säuren, z.B. mit anorganischen Säuren, wie Mineralsäuren, z.B. Salzsäure, Schwefelsäure oder Phosphorsäure, oder mit geeigneten organischen Carbon- oder Sulfonsäuren, z.B. Trifluoressig-, Essig-, Malein-, Fumar-, Wein-, Zitronen-, Methansulfon- oder 4-Toluolsulfonsäure, sowie mit Aminosäuren, wie Arginin und Lysin, bilden.

Die Verbindungen der Formel I können, z.B. wenn sie mehr saure als basische Gruppen enthalten, z.B. wenn $R^4$ Wasserstoff und $R^3$ Carboxy bedeuten, Metall- oder Ammoniumsalze bilden, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B.

Triethylamin, Hydroxyniederalkylamine, z.B. 2-Hydroxyethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z.B. 4-Aminobenzoesäure-2-diethylaminoethylester, Niederalkylenamine, z.B. 1-Ethylpiperidin, Cycloalkylamine, z.B. Dicyclohexylamin, oder Benzylamine, z.B. N,N'-Dibenzylethylendiamin, ferner Basen vom Pyridintyp, z.B. Pyridin, Collidin oder Chinolin.

Zur Isolierung oder Reinigung können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Die neuen Verbindungen der vorliegenden Erfindung weisen eine Reihe wertvoller pharmakologischer Eigenschaften auf.

Erfindungsgemäss wurde überraschenderweise gefunden, dass die obengenannten Phosphatidylverbindungen der Formel I und ihre pharmazeutisch verwendbaren Salze sowohl zur Prophylaxe als auch zur Therapie von Virusinfektionen hervorragend geeignet sind, wie sich z.B. aus Tierversuchen, wie sie im Beispielteil exemplifiziert sind, ergibt. In diesen Tierversuchen werden Tiere, wie Mäuse oder Meerschweinchen, mit den verschiedensten Virusarten in einer Dosis, die für alle oder die grosse Mehrzahl der unbehandelten (Kontroll)-Tiere letal ist, z.B. $LD_{80-90}$, infiziert und der Infektionsverlauf bei den unbehandelten Kontrolltieren im Vergleich zu Tieren beobachtet, die vor, gleichzeitig mit oder nach der Infektion mit einer der obengenannten Verbindungen oder einem Salz davon behandelt werden.

Dabei zeigt sich, dass ein prophylaktischer Effekt bei Verabreichung der Verbindungen der Formel I schon mehrere Tage bis zu einigen, z.B. vier, Wochen vor der Infektion, und ein therapeutischer Effekt noch bei Verabreichung mehrere Tage, z.B. 1 Woche, nach der Infektion, eintritt.

Bemerkenswert ist auch das breite virale Spektrum, gegen das die obengenannten Verbindungen wirksam sind.

Die Verbindungen der Formel I können insbesondere zur Prophylaxe und Therapie von Krankheiten verwendet werden, die durch die nachstehend näher bezeichneten Viren hervorgerufen werden [zur Nomenklatur vgl. J.L.Melnick, Prog. med. Virol. 26, 214-232 (1980) und 28, 208-221 (1982)]:

DNA-Viren mit kubischer Symmetrie und nacktem Nukleokapsid, DNA-Viren mit umhülltem Virion sowie RNA-Viren mit kubischer und solche mit helikaler Symmetrie des Kapsids.

Bevorzugterweise verwendet man die Verbindungen der Formel I im Falle von DNA-Viren mit umhülltem Virion und kubischer Symmetrie des Kapsids, im Falle von RNA-Viren mit kubischer Symmetrie des Kapsids und nacktem Virion und im Falle von RNA-Viren mit helikaler Symmetrie des Kapsids, in denen die Nukleokapsidhülle bei der Oberflächenmembran gelegen ist, aber auch im Falle von Adenoviridae, Poxviridae und Coronaviridae, wie insbesondere menschlichen Coronaviren.

In erster Linie verwendet man die Verbindungen der Formel I im Falle von Herpesviridae, Picornaviridae und Myxoviren, aber auch im Falle von Mastadenoviren, wie insbesondere menschlichen Adenoviren, im Falle von Chordopoxvirinae, wie hauptsächlich Orthopoxviren, wie insbesondere z.B. Vacciniaviren, im Falle von Reoviridae, vornehmlich (insbesondere menschlichen) Rotaviren, sowie im Falle von Caliciviridae und Rhabdoviridae, wie in erster Linie Vesiculoviren des Menschen sowie von Pferden, Rindern und Schweinen.

Hauptsächlich verwendet man die Verbindungen der Formel I im Falle von Alphaherpesvirinae, wie Varicellaviren, z.B. menschlichen Varicella-Zoster Viren, Rhinoviren, Cardioviren und Orthomyxoviridae, aber auch im Falle von Betaherpesvirinae, wie insbesondere menschlichen Cytomegaloviren, im Falle von Aphthoviren, in erster

Linie Apthoviren von Paarhufern, wie hauptsächlich von Rindern, sowie im Falle von Paramyxoviridae, wie in erster Linie Pneumoviren, z.B. respiratorischen Syncitialviren des Menschen, und wie daneben Morbilliviren oder Paramyxoviren, wie Parainfluenzaviren, z.B. menschlichen Parainfluenzaviren, einschliesslich der Sendaiviren sowie im Falle von Arboviren oder Vesiculoviren, z.B. Vesicular stomatitis Viren.

In allererster Linie verwendet man die Verbindungen der Formel I im Falle von Simplexviren, z.B. menschlichen Herpes simplex Viren der Typen 1 und 2, im Falle von menschlichen Encephalomyocarditisviren, im Falle von Influenzaviren, wie hauptsächlich Influenza A und Influenza B Viren und ganz besonders im Falle der in den Beispielen genannten Viren.

Die Verbindungen der Formel I können erfindungsgemäss verwendet werden, indem man sie enteral oder parenteral, in erster Linie zusammen mit geeigneten Hilfs- oder Trägerstoffen, appliziert. Bevorzugterweise appliziert man sie auf die Schleimhaut, z.B. intranasal, rektal, vaginal oder auf die Bindehaut des Auges, oder oral. Der antivirale Effekt tritt jedoch auch bei Applikation auf anderen Wegen, z.B. subkutan, intravenös, intramuskulär oder bei Applikation auf die normale Haut ein.

Die Dosierung des Wirkstoffes hängt u.a. von der Warmblüterspezies, der Abwehrlage des Organismus, der Applikationsweise und der Art des Virus ab. Die Dosis-Wirkungsbeziehung ist relativ schwach ausgeprägt.

Zur Vorbeugung appliziert man eine einmalige Dosis von etwa 0,01 mg bis etwa 25 mg, vorzugsweise 0,05 bis 7 mg, z.B. 0,5 mg, Wirkstoff an einen Warmblüter von etwa 70 kg Körpergewicht, z.B. den Menschen. Die prophylaktische Wirkung dieser Dosis erstreckt sich über mehrere Wochen. Bei Bedarf, z.B. in Zeiten erhöhter Ansteckungsgefahr, kann man die Verabreichung dieser Dosis wiederholen.

Die therapeutische Dosis für Warmblüter von etwa 70 kg Körpergewicht liegt zwischen 0,1 mg und 50 mg, vorzugsweise zwischen 1 und 10 mg, z.B. bei 5 mg, insbesondere bei oraler Applikation. Die Dosierung bei topischer, insbesondere intranasaler Applikation, liegt bis zum Faktor 10 niedriger. Bei Bedarf kann man die Verabreichung der Verbindungen der Formel I bis zum Eintritt einer Besserung der Erkrankung wiederholen. Oft genügt jedoch eine einmalige Applikation.

Die Erfindung betrifft insbesondere die Verwendung von Verbindungen der Formel I, worin $R^1$ und $R^2$ unabhängig voneinander Wasserstoff oder $C_{1-2}$-Alkyl, $R^3$ Wasserstoff, Carboxy oder Benzyloxycarbonyl, $R^4$ Wasserstoff, Benzyl, 2-Amino-ethyl, 2,3-Dihydroxy-propyl, Trimethylsilyl, 2,3,4,5,6-Pentahydroxy-cyclohexyl oder 2-Hydroxy-3-glycyloxypropyl, W Wasserstoff und Z eine 1,2-Dihydroxyethyl- oder 2-Hydroxyethylgrupe, in der jeweils die 2-Hydroxygruppe mit einer geradkettigen Alkansäure mit 6, 10, 12, 14, 16, 18 oder 20 C-Atomen oder mit einer geradkettigen, $C_{18}$-Alkensäure mit 1-3 Doppelbindungen verestert oder mit einem geradkettigen Alkanol mit 6, 8, 16 oder 18 C-Atomen verethert ist und in der die 1-Hydroxygruppe, falls vorhanden, entweder frei oder mit einer geradkettigen Alkansäure mit 6, 10, 12, 14, 16, 18 oder 20 C-Atomen oder mit einer geradkettigen $C_{18}$-Alkensäure mit 1-2 Doppelbindungen verestert oder mit einem geradkettigen Alkanol mit 1, 6, 8, 16 oder 18 C-Atomen verethert ist, oder W Hydroxymethyl oder Palmitoyloxymethyl und Z Palmitoyloxymethyl bedeuten, und von pharmazeutisch verwendbaren Salzen dieser Verbindungen zur Prophylaxe und Therapie von Virusinfektionen bei Warmblütern, wie insbesondere beim Menschen.

Besonders betrifft die Erfindung die Verwendung der Verbindungen der Formel I, worin $R^3$ Wasserstoff, Niederalkoxycarbonyl oder Carboxy und $R^4$ Wasserstoff bedeuten, und ihrer pharmazeutisch verwendbaren Salze zur Prophylaxe und Therapie von Virusinfektionen.

Hauptsächlich betrifft die Erfindung die Verwendung von Verbindungen der Formel I, worin $R^1$ und $R^2$ Wasserstoff, $R^3$ Wasserstoff oder Carboxy, $R^4$ Wasserstoff, W Wasserstoff und Z eine 1,2-Dihydroxy-ethylgruppe, in der die 2-Hydroxygruppe mit einer geradkettigen $C_{14-18}$-Alkansäure mit gerader Anzahl von C-Atomen oder mit einer geradkettigen, einfach ungesättigten $C_{18}$-Alkensäure verestert oder mit einem geradkettigen Alkanol mit 16 oder 18 C-Atomen verethert ist und in der die 1-Hydroxygruppe entweder frei oder mit einer geradkettigen $C_{14-18}$-Alkansäure mit gerader Anzahl von C-Atomen oder mit einer geradkettigen, einfach ungesättigten $C_{18}$-Alkensäure verestert oder mit einem geradkettigen Alkanol mit 1, 16 oder 18 C-Atomen verethert ist, bedeuten, und ihrer pharmazeutisch verwendbaren Salze.

Bevorzugt ist die Verwendung von Verbindungen der Formel I, worin W Wasserstoff und Z eine 1,2-Dihydroxy-ethylgruppe, in der die 2-Hydroxygruppe mit einer geradkettigen $C_{14-18}$-Alkansäure mit gerader Anzahl von C-Atomen oder mit einer geradkettigen, einfach ungesättigten $C_{18}$-Alkensäure verestert ist und in der die 1-Hydroxygruppe entweder frei oder mit einer geradkettigen $C_{14-18}$-Alkansäure mit gerader Anzahl von C-Atomen oder mit einer geradkettigen, einfach ungesättigten $C_{18}$-Alkensäure verestert ist, bedeuten, und ihrer pharmazeutisch verwendbaren Salze.

Besonders bevorzugt ist die Verwendung von Verbindungen der Formel I, worin W Wasserstoff und Z eine 1,2-Dihydroxyethylgruppe, in der die beiden Hydroxygruppen mit einer geradkettigen $C_{14-18}$-Alkansäure mit gerader Anzahl von C-Atomen oder mit einer geradkettigen, einfach ungesättigten $C_{18}$-Alkensäure verestert sind,oder W und Z je eine Hydroxymethylgruppe, die mit einer geradkettigen $C_{14-18}$-Alkansäure mit gerader Anzahl von C-Atomen oder mit einer geradkettigen, einfach ungesättigten $C_{18}$-Alkensäure verestert ist, bedeuten, und ihrer pharmazeutisch verwendbaren Salze.

Ganz besonders betrifft die Erfindung die Verwendung der in den nachstehenden Tabellen 1 bis 3 sowie im Beispielteil aufgeführten Verbindungen und ihrer pharmazeutisch verwendbaren Salze.

In den Tabellen 1 bis 3 werden folgende Abkürzungen verwendet:

| Abkürzungen | |
|---|---|
| Ara | n-Eicosanoyl |
| Capri | Caprinoyl (n-Decanoyl) |
| Capro | Capronyl (n-Hexanoyl) |
| Capry | Capryloyl (n-Octanoyl) |
| Ela | trans-9-Octadecenoyl |
| Laur | Lauroyl |
| Lin | Linoleoyl (9,12-Octadecadienoyl) |
| Linolen | Linolenoyl (9,12,15-Octadecatrienoyl) |
| Myr | Myristoyl |
| Ole | Oleoyl |
| Palm | Palmitoyl |
| Stea | Stearoyl |

Tabelle 1

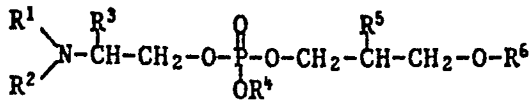

(II)

| $R^1$ | $R^2$ | $R^3$ | $R^4$ | $R^5$ | $R^6$ |
|---|---|---|---|---|---|
| H | H | H | H | O-Capri | Capri |
| H | H | H | H | O-Capry | Capry |
| H | H | H | H | O-Capro | Capro |
| H | H | H | H | O-Laur | Laur |
| H | H | H | H | O-Myr | Myr |
| H | H | H | H | O-Palm | Palm |
| H | H | H | H | O-Stea | Stea |
| H | H | H | H | O-Ara | Ara |
| H | H | H | H | O-Stea | n-$C_{16}H_{33}$ |
| H | H | H | H | O-Myr | n-$C_{16}H_{33}$ |
| H | H | H | H | O-Laur | n-$C_{16}H_{33}$ |
| H | H | H | H | O-Stea | n-$C_{18}H_{37}$ |
| H | H | H | H | O-Myr | n-$C_{18}H_{37}$ |
| H | H | H | H | O-Laur | n-$C_{18}H_{37}$ |
| $CH_3$ | H | H | H | O-Laur | Laur |
| $CH_3$ | H | H | H | O-Myr | Myr |
| $CH_3$ | H | H | H | O-Palm | Palm |
| $CH_3$ | H | H | H | O-Stea | Stea |
| $CH_3$ | H | H | H | O-Ara | Ara |
| $CH_3$ | H | H | H | O-n-$C_{16}H_{33}$ | n-$C_{16}H_{33}$ |
| $CH_3$ | $CH_3$ | H | H | O-Laur | Laur |

Tabelle 1 (Fortsetzung)

| $R^1$ | $R^2$ | $R^3$ | $R^4$ | $R^5$ | $R^6$ |
|---|---|---|---|---|---|
| $CH_3$ | $CH_3$ | H | H | O-Myr | Myr |
| $CH_3$ | $CH_3$ | H | H | O-Palm | Palm |
| $CH_3$ | $CH_3$ | H | H | O-Stea | Stea |
| $CH_3$ | $CH_3$ | H | H | O-Ara | Ara |
| $CH_3$ | $CH_3$ | H | H | O-n-$C_{16}H_{33}$ | n-$C_{16}H_{33}$ |
| $CH_3$ | $CH_3$ | H | H | O-Palm | Ole |
| H | H | H | H | OH | Palm |
| $CH_3$ | $CH_3$ | H | H | OH | Palm |
| H | H | H | H | H | Laur |
| H | H | H | H | H | Myr |
| H | H | H | H | H | Palm |
| H | H | H | H | H | Stea |
| H | H | H | H | H | Ara |
| $CH_3$ | H | H | H | H | Laur |
| $CH_3$ | H | H | H | H | Myr |
| $CH_3$ | H | H | H | H | Palm |
| $CH_3$ | H | H | H | H | Stea |
| $CH_3$ | H | H | H | H | Ara |
| $CH_3$ | $CH_3$ | H | H | H | Laur |
| $CH_3$ | $CH_3$ | H | H | H | Myr |
| $CH_3$ | $CH_3$ | H | H | H | Palm |
| $CH_3$ | $CH_3$ | H | H | H | Stea |
| $CH_3$ | $CH_3$ | H | H | H | Ara |
| H | H | H | H | O-n-$C_{16}H_{33}$ | n-$C_{16}H_{33}$ |

Tabelle 1 (Fortsetzung)

| $R^1$ | $R^2$ | $R^3$ | $R^4$ | $R^5$ | $R^6$ |
|---|---|---|---|---|---|
| H | H | H | H | $OCH_3$ | n-$C_{16}H_{33}$ |
| $CH_3$ | $CH_3$ | H | H | O-n-$C_8H_{17}$ | n-$C_8H_{17}$ |
| $CH_3$ | $CH_3$ | H | H | O-n-$C_6H_{13}$ | n-$C_6H_{13}$ |
| H | H | H | H | OH | Laur |
| H | H | H | H | OH | Myr |
| $CH_3$ | $C_2H_5$ | H | H | H | Laur |
| $C_2H_5$ | $C_2H_5$ | H | H | H | Laur |
| $CH_3$ | $C_2H_5$ | H | H | H | Myr |
| $C_2H_5$ | $C_2H_5$ | H | H | H | Myr |
| $CH_3$ | $C_2H_5$ | H | H | H | Palm |
| $C_2H_5$ | $C_2H_5$ | H | H | H | Palm |
| H | H | H | H | O-Ela | Ela |
| H | H | H | H | O-Ole | Palm |
| $CH_3$ | $CH_3$ | H | H | O-Ole | Palm |
| $CH_3$ | $C_2H_5$ | H | H | O-Myr | Myr |
| $CH_3$ | $C_2H_5$ | H | H | O-Palm | Palm |
| $CH_3$ | $C_2H_5$ | H | H | O-Stea | Stea |
| $C_2H_5$ | $C_2H_5$ | H | H | O-Myr | Myr |
| $C_2H_5$ | $C_2H_5$ | H | H | O-Palm | Palm |
| $C_2H_5$ | $C_2H_5$ | H | H | O-Stea | Stea |
| H | H | H | H | O-Palm | n-$C_{16}H_{33}$ |
| H | H | H | H | O-Palm | n-$C_{18}H_{37}$ |
| H | H | COOH | $-CH_2CH_2-CH_2$ | O-Palm | Palm |

Tabelle 1 (Fortsetzung)

| $R^1$ | $R^2$ | $R^3$ | $R^4$ | $R^5$ | $R^6$ |
|---|---|---|---|---|---|
| H | H | H | $-CH_2-CH-CH_2OH$<br>OH | O-Palm | Palm |
| H | H | H | $Si(CH_3)_3$ | O-Palm | Palm |
| H | H | H | 2,3,4,5,6-Pentahydroxy-cyclohexyl | O-Stea | Stea |
| H | H | H | Benzyl | O-Palm | Palm |
| H | H | H | OH O<br>$CH_2-CH-CH_2O-C-CH_2-NH_2$ | O-Stea | Stea |
| H | H | COOH | H | O-Ole | Ole |
| H | H | COOH | H | O-Lin | Lin |
| H | H | COOH | H | O-Myr | Myr |
| H | H | COOH | H | O-Capro | Capro |
| H | H | COOH | H | O-Capri | Capri |
| H | H | COOH | H | O-Laur | Laur |
| H | H | COOH | H | O-Palm | Palm |
| H | H | COOH | H | O-Stea | Stea |
| H | H | COOH | H | O-Palm | Ole |
| H | H | COOH | H | O-Palm | Lin |
| H | H | COOH | H | O-Palm | Linolen |

Tabelle 2

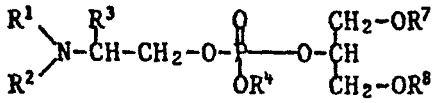

(III)

| $R^1$ | $R^2$ | $R^3$ | $R^4$ | $R^7$ | $R^8$ |
|---|---|---|---|---|---|
| H | H | H | H | Palm | Palm |
| $CH_3$ | H | H | H | Palm | Palm |
| $CH_3$ | $CH_3$ | H | H | Palm | Palm |
| H | H | H | H | Palm | H |
| H | H | H | H | H | Palm |

Tabelle 3

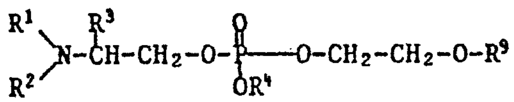

(IV)

| $R^1$ | $R^2$ | $R^3$ | $R^4$ | $R^9$ |
|---|---|---|---|---|
| H | H | H | H | Laur |
| H | H | H | H | Myr |
| H | H | H | H | Palm |
| $CH_3$ | H | H | H | Palm |
| $CH_3$ | $CH_3$ | H | H | Palm |
| H | H | H | H | Stea |

Die Erfindung betrifft auch Kephalinderivate der Formel I,

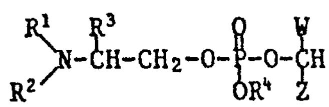

(I)

worin $R^1$ und $R^2$ unabhängig voneinander Wasserstoff oder Niederalkyl, $R^3$ Wasserstoff, Niederalkoxycarbonyl, Carbamoyl oder freies oder geschütztes Carboxy, $R^4$ Wasserstoff oder einen aliphatischen, aromatischen, aromatisch-aliphatischen oder cycloaliphatischen Rest, W Wasserstoff und Z eine 1,2-Dihydroxy-ethyl-, 2-Hydroxy-ethyl-, oder Hydroxymethyl-Gruppe, in der mindestens eine Hydroxygruppe mit

einer aliphatischen $C_{6-30}$-Carbonsäure verestert oder mit einem aliphatischen $C_{6-30}$-Alkohol verethert ist, und in der die andere Hydroxygruppe, falls vorhanden, frei, mit einer aliphatischen $C_{2-30}$-Carbonsäure verestert oder mit einem aliphatischen $C_{1-30}$-Alkohol verethert ist, oder W Hydroxymethyl oder eine Hydroxymethylgruppe, die mit einer aliphatischen $C_{6-30}$-Carbonsäure verestert oder mit einem aliphatischen $C_{6-30}$-Alkohol verethert ist, und Z eine Hydroxymethylgruppe, die mit einer aliphatischen $C_{6-30}$-Carbonsäure verestert oder mit einem aliphatischen $C_{6-30}$-Alkohol verethert ist, bedeuten, wobei $R^1$ von Wasserstoff und $C_{1-2}$-Alkyl, oder $R^2$ von Wasserstoff und $C_{1-2}$-Alkyl, oder $R^3$ von Wasserstoff, Carboxy und Benzyloxycarbonyl, oder $R^4$ von Wasserstoff, Benzyl, 2-Amino-ethyl, 2,3-Dihydroxy-propyl, Trimethylsilyl, 2,3,4,5,6-Pentahydroxy-cyclohexyl und 2-Hydroxy-3-glycyloxy-propyl, oder, falls W für Wasserstoff steht, Z von einer 1,2-Dihydroxyethyl- und 2-Hydroxy-ethylgruppe, in der jeweils die 2-Hydroxygruppe mit einer geradkettigen Alkansäure mit 6, 10, 12, 14, 16, 18 oder 20 C-Atomen oder mit einer geradkettigen $C_{18}$-Alkensäure mit 1-3 Doppelbindungen verestert oder mit einem geradkettigen Alkanol mit 6, 8, 16 oder 18 C-Atomen verethert ist und in der die 1-Hydroxygruppe, falls vorhanden, entweder frei oder mit einer geradkettigen Alkansäure mit 6, 10, 12, 14, 16, 18 oder 20 C-Atomen oder mit einer geradkettigen $C_{18}$-Alkensäure mit 1-2 Doppelbindungen verestert oder mit einem geradkettigen Alkanol mit 1, 6, 8, 16 oder 18 C-Atomen verethert ist, oder, falls W nicht für Wasserstoff steht, W von Palmitoyloxymethyl und Hydroxymethyl, oder, falls W nicht für Wasserstoff steht, Z von Palmitoyloxymethyl verschieden ist, und Salze dieser Verbindungen.

Insbesondere betrifft die Erfindung Verbindungen der Formel I, worin $R^1$ und $R^2$ unabhängig voneinander Wasserstoff oder Niederalkyl, $R^3$ Wasserstoff, Carboxy oder Niederalkoxycarbonyl, $R^4$ Wasserstoff und W und Z je mit einer geradkettigen $C_{6-22}$-Alkansäure, die von Palmitinsäure verschieden ist, oder mit einer geradkettigen $C_{18}$-Alkensäure mit 1-3 Doppelbindungen verestertes Hydroxymethyl bedeuten, und ihre pharmazeutisch verwendbaren Salze.

worin die Substituenten die obengenannten Bedeutungen haben, oder mit einem reaktionsfähigen Derivat davon umsetzt und, wenn erforderlich, vorhandene Schutzgruppen abspaltet, oder

c) eine Verbindung der Formel VIII,

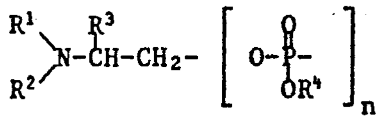 (VIII)

worin n die untengenannte Bedeutung hat, und die übrigen Substituenten die obengenannten Bedeutungen haben, wobei in einer Verbindung der Formel VIII vorhandene funktionelle Gruppen mit Ausnahme der an der Reaktion teilnehmenden Gruppe erforderlichenfalls in geschützter Form vorliegen, oder ein reaktionsfähiges Derivat davon mit einer Verbindung der Formel IX,

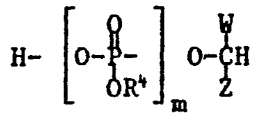 (IX)

worin m für 1 steht, wenn im Reaktionspartner der Formel VIII n für 0 steht, oder worin m für 0 steht, wenn n für 1 steht, und die übrigen Substituenten die obengenannten Bedeutungen haben, wobei in einer Verbindung der Formel IX vorhandene funktionelle Gruppen mit Ausnahme der an der Reaktion teilnehmenden Gruppe erforderlichenfalls in geschützter Form vorliegen, oder mit einem reaktionsfähigen Derivat davon umsetzt und, wenn erforderlich, vorhandene Schutzgruppen abspaltet, oder

d) eine Verbindung der Formel X,

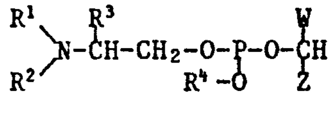 (X)

worin die Substituenten die obengenannten Bedeutungen haben, wobei in einer Verbindung der Formel X vorhandene funktionelle Gruppen mit Ausnahme der an der Reaktion teilnehmenden Gruppe erforderlichen-Hauptsächlich betrifft die Erfindung die letztgenannten Verbindungen der Formel I, worin $R^2$ von Methyl verschiedenes Niederalkyl und/oder $R^3$ Carboxy oder Niederalkoxycarbonyl bedeuten, und die übrigen Substituenten die im vorangehenden Abschnitt genannten Bedeutungen haben, und ihre pharmazeutisch verwendbaren Salze.

Die Kephalinderivate der Formel I und ihre Salze werden nach an sich bekannten Methoden hergestellt. Sie werden z.B. erhalten, indem man

a) zur Herstellung einer Verbindung der Formel I, worin mindestens einer der Reste $R^1$ und $R^2$ Niederalkyl bedeutet, eine Verbindung der Formel V,

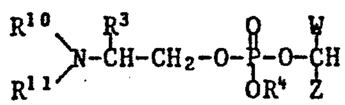
(V)

worin mindestens einer der Reste $R^{10}$ und $R^{11}$ für Wasserstoff steht und der andere der Reste $R^{10}$ und $R^{11}$ Wasserstoff oder Niederalkyl bedeutet, und die übrigen Substituenten die obengenannten Bedeutungen haben, wobei in einer Verbindung der Formel V vorhandene funktionelle Gruppen mit Ausnahme der an der Reaktion teilnehmenden Gruppe erforderlichenfalls in geschützter Form vorliegen, alkyliert, und, wenn erforderlich, vorhandene Schutzgruppen abspaltet, oder

b) eine Verbindung der Formel VI,

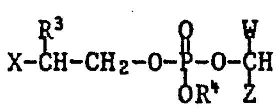
(VI)

worin X für eine nucleophile Abgangsgruppe steht, und die übrigen Substituenten die obengenannten Bedeutungen haben, wobei in einer Verbindung der Formel VI vorhandene funktionelle Gruppen mit Ausnahme der an der Reaktion teilnehmenden Gruppe erforderlichenfalls in geschützter Form vorliegen, mit einer Verbindung der Formel VII,

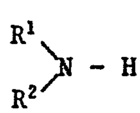
(VII)

falls in geschützter Form vorliegen, oder ein Tautomeres einer Verbindung der Formel X mit einem Oxidationsmittel oxidiert und, wenn erforderlich, vorhandene Schutzgruppen abspaltet, oder

e) eine Verbindung der Formel XI,

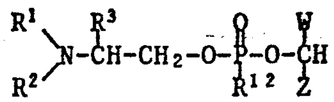

(XI)

worin $R^{12}$ Halogen bedeutet, und die übrigen Substituenten die obengenannten Bedeutungen haben, wobei in einer Verbindung der Formel XI vorhandene funktionelle Gruppen mit Ausnahme der an der Reaktion teilnehmenden Gruppe erforderlichenfalls in geschützter Form vorliegen, hydrolysiert und, wenn erforderlich, vorhandene Schutzgruppen abspaltet, oder

f) eine Verbindung der Formel XII,

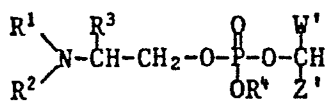

(XII)

worin W' Wasserstoff und Z' 1,2-Dihydroxy-ethyl, 2-Hydroxy-ethyl, Hydroxymethyl oder 1,2-Dihydroxy-ethyl, worin eine der beiden Hydroxygruppen wie oben beschrieben verestert oder verethert ist oder mindestens einer der Reste W' und Z' freies Hydroxymethyl und der andere der Reste W' und Z' freies Hydroxymethyl oder Hydroxymethyl, das wie oben beschrieben verestert oder verethert ist, bedeuten, und die übrigen Substituenten die obengenannten Bedeutungen haben, wobei in einer Verbindung der Formel XII vorhandene funktionelle Gruppen mit Ausnahme der an der Reaktion teilnehmenden Gruppe erforderlichenfalls in geschützter Form vorliegen, oder ein reaktionsfähiges Derivat davon mit einer aliphatischen Carbonsäure oder einem reaktionsfähigen Derivat davon verestert oder mit einem aliphatischen Alkohol oder einem reaktionsfähigen Derivat davon verethert und, wenn erforderlich, vorhandene Schutzgruppen abspaltet, oder

g) zur Herstellung einer Verbindung der Formel I, worin W Wasserstoff und Z eine 1,2-Dihydroxy-ethylgruppe bedeutet, worin die 2-Hydroxygruppe mit einer aliphatischen $C_{6-30}$-Carbonsäure verestert ist, und $R^1$, $R^2$, $R^3$ und $R^4$ die obengenannten Bedeutungen haben, eine Verbindung der Formel I, worin W Wasserstoff und Z eine 1,2-Dihydroxy-ethylgruppe bedeutet, worin die 2-Hydroxygruppe mit einer aliphatischen $C_{6-30}$-Carbonsäure und die 1-Hydroxygruppe mit einer aliphatischen $C_{2-30}$-Carbonsäure verestert ist, und $R^1$, $R^2$, $R^3$ und $R^4$ die obengenannten Bedeutungen haben, mit einem Enzym umsetzt, das die 1-Acylgruppe regioselektiv abspaltet, oder

h) in einer Verbindung der Formel I, worin die Substituenten die obengenannten Bedeutungen haben, wobei in einer Verbindung der Formel I mindestens eine funktionelle Gruppe durch eine leicht abspaltbare Schutzgruppe geschützt sein muss, die Schutzgruppe(n) abspaltet

und nach Ausführung einer der obengenannten Verfahrensvarianten a-h) zur Herstellung eines Salzes erforderlichenfalls eine Verbindung der Formel I in ein Salz überführt oder zur Ueberführung einer Verbindung der Formel I oder eines Salzes davon in eine andere Verbindung der Formel I oder in ein Salz davon eine im Rest $R^3$ enthaltene Carboxylgruppe verestert oder amidiert.

Die obengenannten Verfahrensvarianten werden im folgenden näher erläutert:

Allgemeines:
Funktionelle Gruppen, die erforderlichenfalls geschützt sind, sind in erster Linie Carboxy, Hydroxy, Amino und/oder die Phosphorsäuregruppe.

Carboxylschutzgruppen sind z.B. die obengenannten.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa-oder 2-thia-aliphatische oder -cycloaliphatische Kohlenwasserstoffreste, in erster Linie 1-Niederalkoxyniederalkyl oder 1-Niederalkylthio-niederalkyl, z.B. Methoxymethyl, 1-Methoxy-ethyl, 1-Ethoxy-ethyl, Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere Trimethylsilyl, ferner Dimethyl-tert.-butyl-silyl, oder entsprechend substituiertes Stannyl, z.B. Tri-n-butylstannyl.

Eine geschützte Aminogruppe kann z.B. in Form einer leicht spaltbaren Acylamino-, Arylmethylamino-, veretherten Mercaptoamino-, 2-Acyl-niederalk-1-en-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Iod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.Butyl, Niederalkoxy, wie Methoxy, Hydroxy, Halogen, z.B. Chlor, und/oder Nitro,mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit bis zu 15 C-Atomen, wie entsprechendes,

gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-(4-nitrobenzyl)-phosphoryl, gegebenenfalls substituiertes Phenyloxy-phenyl-phosphonyl, z.B. Phenyloxyphenyl-phosphonyl, Diniederalkylphosphinyl, z.B. Diethylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-enl-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie

Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Nieder-alkanoyl-prop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en-2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl.

Die Phosphorsäuregruppe ist vorzugsweise in veresterter Form geschützt, wobei die veresternden Reste vorzugsweise aliphatischer, aromatischer oder aromatisch-aliphatischer Natur sind, z.B. Niederalkyl, wie insbesondere Methyl, gegebenenfalls, z.B. durch Nitro oder Halogen, substituiertes Phenyl, oder gegebenenfalls, z.B. durch Nitro oder Halogen, substituiertes Benzyl.

Die Abspaltung der obengenannten Schutzgruppen wird z.B. wie unten bei Verfahren g beschrieben durchgeführt.

Verfahren a:

Die Alkylierung der Aminogruppe in einer Verbindung der Formel V wird z.B. mit einem der nachstehend genannten Mittel durchgeführt.

Geeignete Mittel sind beispielsweise entsprechende Diazoverbindungen, wie gegebenenfalls substituierte Diazoniederalkane, z.B. Diazomethan, Diazoethan, Diazo-n-butan oder Diphenyldiazomethan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Ethers, wie eines Diniederalkylethers, z.B. Diethylether, oder eines cyclischen Ethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Mittel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Iodwasserstoffsäure, ferner Schwefelsäure, oder Halogen-schwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester, z.B. Fluorsulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Ethers, wie Dioxan oder Tetrahydrofuran, oder eines Gemisches davon verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin (vorzugsweise zusammen mit Halogensulfonsäure-niederalkylestern oder gegebenenfalls halogensubstituierten Methansulfonsäureniederalkylestern) an, wobei unter Kühlen, bei Raumtemperatur oder unter Erwärmen, z.B. bei Temperaturen von etwa -20°C bis etwa 50°C, und, wenn notwendig, in einem geschlossenen Gefäss und/oder in einer Inertgas-, z.B. Stickstoffatmosphäre, gearbeitet wird.

Weitere Mittel sind entsprechende trisubstituierte Oxoniumsalze (sogenannte Meerweinsalze), oder disubstituierte Carbenium- oder Haloniumsalze, worin die Substituenten die verethernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z.B. Trimethyloxonium- oder Triethyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Mittel vorzugsweise in einem inerten Lösungsmittel, wie einem Ether oder einem halogenierten Kohlenwasserstoff, z.B. Diethylether, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-ethyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Verfahren b:

Eine nucleophile Abgangsgruppe X ist insbesondere mit geeigneten Säuren verestertes Hydroxy.

Reaktionsfähiges verestertes Hydroxy ist z.B. mit einer starken anorganischen oder organischen Säure verestertes Hydroxy, wie mit einer Mineralsäure, z.B. Halogenwasserstoffsäure, wie Chlorwasserstoff-, Bromwasserstoff- oder Iodwasserstoffsäure, ferner Schwefelsäure, oder Halogenschwefelsäure, z.B. Fluorschwefelsäure, oder mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom, und/oder Nitro substituierten Benzolsulfonsäure, z.B. einer Methansulfon-, Trifluormethansulfon- oder p-Toluolsulfonsäure verestertes Hydroxy, bevorzugterweise ein Chlorid, Bromid oder Iodid.

Verfahren c:
Ein reaktionsfähiges Derivat einer Verbindung der Formel VIII, worin n für 1 steht, oder einer Verbindung der Formel IX, worin m für 1 steht, ist z.B. ein Mono- oder Bisanhydrid mit einer starken Säure, insbesondere einer Mineralsäure, wie vornehmlich einer Halogenwasserstoffsäure, wie hauptsächlich Chlorwasserstoffsäure. Die zweite saure Phosphorsäuregruppe kann als solche, als Anhydrid wie oben geschildert oder in veresterter Form vorliegen, wobei als veresternde Reste solche bevorzugt sind, die nach erfolgter Reaktion zwischen den Verbindungen VIII und IX regioselektiv wieder abgespalten werden können, z.B. die Methylestergruppe, die z.B. mittels Lithiumbromid abgespalten werden kann, oder elektrochemisch abspaltbare Reste, z.B. Benzyl- oder Phenylesterreste.

Die Bildung reaktionsfähiger Phosphorsäurederivate kann auch in situ in Gegenwart von Verbindungen erfolgen, die mit Phosphorsäure oder deren Monoestern zumindest intermediär reaktionsfähige Verbindungen mit anhydrid- oder enolesterartigem Charakter einzugehen vermögen, z.B. in Gegenwart von p-Toluolsulfonsäurechlorid, Cyanurchlorid, N-Alkyl-5-phenylisoxazoliumsalzen, Ethoxyacetylen oder bevorzugterweise Trichloracetonitril oder insbesondere einem Carbodiimid, wie hauptsächlich Dicyclohexylcarbodiimid. Beispielsweise kann man einen Phosphorsäuremonoester der Formeln VIII oder IX, worin n bzw. m für

1 stehen, mit überschüssigem Alkohol der Formeln IX bzw. VIII, worin n bzw. m für 0 stehen, in Gegenwart der mehrfachen, z.B. fünffachen, molaren Menge von Dicyclohexylcarbodiimid in An- oder Abwesenheit eines tertiären Amins umsetzen.

Wenn in einem Phosphorsäuremonoester beide sauren Gruppen als Anhydrid mit einer Halogenwasserstoffsäure vorliegen, kann man zunächst neben dem Triester auch Phosphorsäurediester-halogenide erhalten, die anschliessend durch Wasser, wasserabgebende Mittel oder durch Erhitzen mit tertiären Alkoholen, wie tert.-Butanol oder Tetrahydropyranol, zu Diestern hydrolysiert werden können.

Wenn man von einem Phosphorsäuremonoester-dihalogenid, z.B. einem Phosphorsäuremonoester-dichlorid, ausgeht, führt man die Umsetzung bevorzugterweise in Anwesenheit eines tertiären Amins, wie Triethylamin, Pyridin, Lutidin oder Chinolin durch, wobei eine zusätzliche Aktivierung des Esterchlorids durch Dimethylformamid bewirkt wird.

Eine bevorzugte Ausführungsform des Verfahrens c ist die Umsetzung eines Phosphorsäuremonoester-dichlorids mit dem entsprechenden Alkohol in Gegenwart eines tertiären Amins, gefolgt von der Hydrolyse des zunächst erhaltenen Phosphorsäurediesterhalogenids.

In einem reaktionsfähigen Derivat einer Verbindung der Formeln VIII oder IX, worin n bzw. m für 0 stehen, liegt die an der Reaktion teilnehmende Hydroxygruppe in reaktionsfähiger, veresterter Form vor.

Reaktionsfähiges verestertes Hydroxy ist z.B. wie bei Verfahren b) beschrieben verestertes Hydroxy.

Die Reaktion kann so durchgeführt werden, dass man ein reaktionsfähiges Phosphorsäurederivat der Formeln VIII oder IX mit einem Alkohol der Formeln IX bzw. VIII in unaktivierter Form umsetzt, oder indem man einen reaktionsfähigen veresterten Alkohol der

Formeln VIII oder IX mit einem Phosphorsäurederivat der Formeln IX bzw. VIII in unaktivierter Form oder einem reaktionsfähigen Salz davon umsetzt.

Als Salze von Verbindungen der Formeln VIII oder IX verwendet man im Hinblick auf die beabsichtigte nucleophile Substitutionsreaktion besonders reaktionsfähige Salze, z.B. Salze, wie Silbersalze, die mit der nucleophilen Abgangsgruppe im Reaktionspartner, z.B. einem der obengenannten Halogenidionen, einen schwerlöslichen Niederschlag zu bilden vermögen, oder Salze mit grossem Kation, z.B. Cäsiumsalze, in denen die Nucleophilie des Phosphatrestes erhöht ist. Zur Erhöhung der Nucleophilie des Phosphatrestes kann das Gegenion auch räumlich entfernt werden, z.B. durch Zugabe von Komplexbildnern, wie Kronenethern, z.B. 18-Krone-6. Bei Verwendung von 18-Krone-6 kann man die Reaktion mit einem Kaliumsalz durchführen.

Eine bevorzugte Ausführungsform des Verfahrens b ist die Umsetzung des Cäsium- oder Silbersalzes eines Phosphorsäuremonoesters der Formeln VIII oder IX, worin eine der beiden sauren Gruppen durch eine leicht abspaltbare Schutzgruppe, z.B. eine der oben beschriebenen, geschützt ist, z.B. als Benzyl- oder Phenylester, mit einem reaktionsfähigen Alkohol der Formel IX bzw. VIII, worin die OH-Gruppe durch Chlor, Brom oder Iod ersetzt ist. Nach erfolgter Umsetzung wird die Schutzgruppe abgespalten, z.B. eine Benzyl- oder Phenylesterschutzgruppe wie oben beschrieben.

Verfahren d:
Die Verbindungen der Formel X, worin $R^4$ für Wasserstoff steht, liegen zum überwiegenden Teil in der tautomeren Form vor, worin ein Proton direkt an Phosphor gebunden ist. Die Oxidation kann z.B. mit wässerigem Kaliumpermanganat bei Temperaturen um 0°C durchgeführt werden. In wässerigem Medium sind auch Alkalijodate, -perjodate und -hypochlorite, Peressigsäure, N-Chlor-4-methyl-benzol-sulfonsäureamid u.a. als Oxidationsmittel geeignet.

Verfahren e:
In einer Verbindung der Formel XI steht $R^{12}$ für ein Halogen, wie Brom oder Iod, in allererster Linie aber für Chlor.

Die Hydrolyse wird mit Wasser oder einem wasserabgebenden Mittel, vorzugsweise bei erhöhter Temperatur, z.B. zwischen 30 und 95°C, durchgeführt.

Die Ausgangsstoffe sind z.B. wie bei Verfahren c beschrieben oder durch Chlorierung der entsprechenden Phosphorigsäurediester, z.B. mit elementarem Chlor, erhältlich.

Verfahren f:
In einem reaktionsfähigen Derivat einer Verbindung der Formel XII liegt die an der Reaktion teilnehmende Hydroxygruppe in reaktionsfähiger, veresterter Form vor, z.B. wie bei Verfahren b beschrieben.

Reaktionsfähige Carbonsäurederivate sind in erster Linie reaktionsfähige aktivierte Ester oder reaktionsfähige Anhydride, ferner reaktionsfähige cyclische Amide; dabei können reaktionsfähige Carbonsäurederivate auch <u>in situ</u> gebildet werden.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann;

Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. duch Nitro, substituierte Phenyl-thioester (die man z.B. durch Behandeln der entsprechenden Säure mit gegebenenfalls, z. B. durch Nitro, substituierten Thiophenolen, u.a. mit Hilfe der Anhydrid-oder Carbodiimid-Methode, erhalten kann; Methode der aktivierten Thiolester), Amino-oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxyphthalimid oder 1-Hydroxy-benztriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann).

Anhydride von Säuren können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden

Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Wie erwähnt können reaktionsfähige Carbonsäurederivate auch in situ gebildet werden. So kann man z.B. N,N'-disubstituierte Amidinoester in situ bilden, indem man das Gemisch des Ausgangsmaterials der Formel XII und der aliphatischen Carbonsäure in Gegenwart eines geeigneten N,N-disubstituierten Carbodiimids, z.B. N,N'-Dicyclohexylcarbodiimid, zur Reaktion bringt. Ferner kann man Amino- oder

Amidoester der aliphatischen Carbonsäure in Gegenwart des zu acylierenden Ausgangsmaterials der Formel XII bilden, indem man das Gemisch der Ausgangsstoffe in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxysuccinimid, gegebenenfalls in Anwesenheit einer geeigneten Base, z.B. 4-Dimethylaminopyridin, umsetzt.

Ein reaktionsfähiges Derivat des aliphatischen Alkohols ist z.B. ein Alkohol, in dem die Hydroxygruppe in reaktionsfähiger, veresterter Form vorliegt, z.B. wie bei Verfahren b beschrieben. Geeignete Mittel zur Veretherung einer Hydroxygruppe im Rest W' oder Z' sind z.B. auch die bei Verfahren a genannten Mittel. Die Reaktion wird z.B. durchgeführt, indem man eine Verbindung der Formel XII in unaktivierter Form mit dem reaktionsfähigen Carbonsäure- oder Alkoholderivat umsetzt, wobei die Aktivierung der Carbonsäure auch in situ, in Gegenwart der Verbindung der Formel XII, erfolgen kann, z.B. wie oben beschrieben. Alternativ kann die Reaktion durchgeführt werden, indem man eine Verbindung der Formel XII, worin die an der Reaktion teilnehmende(n) Hydroxygruppe(n) in reaktionsfähiger, veresterter Form vorliegt (vorliegen), z.B. als Halogenid, mit einer Carbonsäure oder einem Alkohol in jeweils unaktivierter Form oder mit einem reaktionsfähigen Carbonsäuresalz, z.B. einem Cäsiumsalz, umsetzt.

Verfahren g:
Die Reaktion wird z.B. mit dem Enzym Phospholipase $A_2$ in Gegenwart von Calciumchlorid durchgeführt.

Verfahren h:
Funktionelle Gruppen in einer Verbindung der Formel I, die gegebenenfalls durch eine leicht abspaltbare Schutzgruppe geschützt sind, sind insbesondere eine Aminogruppe $N(R^1,R^2)$, freies Carboxy $R^3$, die Phosphorsäuregruppe oder freies Hydroxy im Rest W oder Z. Schutzgruppen für die genanten funktionellen Gruppen sind z.B. die oben bei "Allgemeines" genannten.

Die Abspaltung der Schutzgruppen erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische oder elektrochemische Reduktion, oder enzymatisch, stufenweise oder gleichzeitig.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Iod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden

Reagens, wie Natriumthiophenolat oder Natriumjodid, gespalten werden kann. Substituiertes 2-Silylethoxycarbonyl kann auch durch Behandeln mit einem, das Fluoridanion liefernden Salz der Fluorwaserstoffsäure, wie einem Alkalimetallfluorid, z.B. Natrium-oder Kaliumfluorid, in Anwesenheit eines macrocyclischen Polyethers ("Kronenether"), oder mit einem Fluorid einer organischen quaternären Base, wie Tetra-niederalkylammoniumfluorid oder Triniederalkyl-aryl-ammoniumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silylgruppe, wie Triniederalkylsilyl z.B. Trimethylsilyl, verestertes Carboxyl kann in üblicher Weise solvolytisch, z.B. durch Behandeln mit Wasser, einem Alkohol oder Säure freigesetzt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfals nach Umwandlung einer 2-Brom-niederalkoxycarbonylaminogruppe in eine 2-Iod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silylethoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure,

wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silylethoxycarbonyl geschützte Aminogruppe kann auch durch Behandeln mit einem Fluoridanionen liefernden Salz der Fluorwaserstoffsäure, wie oben im Zusammenhang mit der Freisetzung einer entsprechend geschützten Carboxylgruppe angegeben, in die freie Aminogruppe übergeführt werden.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxygruppe wird analog einer entsprechend geschützten Aminogruppe freigesetzt. Eine durch 2,2-Dichloracetyl geschützte Hydroxygruppe wird z.B. durch basische Hydrolyse, eine durch tert.-Niederalkyl oder durch einen 2-oxa- oder 2-thia-aliphatischen oder -cycloaliphatischen Kohlenwasserstoffrest veretherte Hydroxygruppe durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

Phosphorsäureniederalkylester, z.B. -methylester, werden vorzugsweise mittels Lithiumbromid, aber auch durch alkalische Verseifung gespalten. Phosphorsäurephenyl- oder -benzylester werden vorzugsweise elektrochemisch gespalten, oder auch hydrogenolytisch, wobei Benzylester z.B. in Gegenwart von Palladiumkatalysatoren, wie Palladium-auf-Kohlenstoff, und Phenylester z.B. in Gegenwart von Platin- oder gemischten Platin-Palladium-Katalysatoren gespalten werden können.

Die oben beschriebenen Verfahren, inklusive die Verfahren zur Abspaltung von Schutzgruppen und die zusätzlichen Verfahrensmassnahmen, werden in an sich bekannter Weise, z.B. in An-oder Abwesenheit von Lösungs- und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa 150°C, in einem geschlossenen Gefäss und/oder in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Gemische von Isomeren können in an sich bekannter Weise, z.B. durch fraktionierte Kristallisation, Chromatographie etc. in die einzelnen Isomeren, Racemate z.B. unter Bildung von Derivaten mit optisch aktiven Verbindungen und Trennung der so erhältlichen Diastereomerengemische in die optisch aktiven Antipoden, aufgetrennt werden.

Die Erfindung betrifft auch neue pharmazeutische Präparate, die als Wirkstoff, vorzugsweise als alleinigen Wirkstoff, eine zur Prophylaxe oder Therapie von Virusinfektionen wirksame Menge einer Verbindung der Formel I, gegebenenfalls zusammen mit pharmazeutisch verwendbaren Trägerstoffen enthalten, die sich zur enteralen, z.B. oralen oder rektalen, oder parenteralen Verabreichung eignen, und anorganisch oder organisch, fest oder flüssig sein können. Diese Präparate können z.B. in Dosiseinheitsform vorliegen. So verwendet man Tabletten oder Gelatinekapseln, welche den Wirkstoff zusammen mit Verdünnungsmitteln, z.B. Lactose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyethylenglykol, enthalten. Tabletten können ebenfalls Bindemittel, z.B. Magnesiumaluminiumsilikat,

Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz-und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z.B. mittels konventioneller Misch-, Granulier-, Dragier-, Lösungs- oder Lyophilisierungsverfahren, hergestellt und enthalten von etwa 0,001 % bis 99 %, insbesondere von etwa 0,01 % bis etwa 10 %, in erster Linie zwischen 0,1 % und 5 %, des bzw. der Aktivstoffe, wobei eine Wirkstoffkonzentration unterhalb von 1 % insbesondere für topisch zu applizierende Präparate geeignet ist.

Als topisch zu applizierende Darreichungsformen sind die folgenden bevorzugt: Crèmes, Salben oder Pasten mit einem Wirkstoffgehalt von 0,001 % bis 1 %, insbesondere von 0,01 % bis 0,1 %, z.B. 0,05 %, z.B. Salben zur intranasalen Applikation oder Lippenstifte, oder wässrige Lösungen mit einem Wirkstoffgehalt von 0,001 % bis 1 %, insbesondere 0,05 % bis 0,5 %, z.B. 0,1 %, vorzugsweise isotonische, sterile und physiologisch verträgliche Lösungen, z.B. Augentropfen, vorzugsweise in Mikrobehältern zur einmaligen Verwendung, oder Sprays zur Anwendung im Mund- und Rachenraum.

Besonders geeignet sind die in den Beispielen beschriebenen pharmazeutischen Präparate.

Crèmes sind Oel-in-Wasser-Emulsionen, die mehr als 50 % Wasser aufweisen. Als ölige Grundlage verwendet man in erster Linie Fettalkohole, z.B. Lauryl-, Cetyl- oder Stearylalkohole, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitan-fettsäureester (Tweens), ferner Polyoxyethylen-fettalkoholether oder -fettsäureester, oder entsprechende ionische Emulgatoren, wie Alkailmetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Crèmes vermindern, z.B. Polyalkohol, wie Glycerin, Sorbit, Propylenglykol und/oder Polyethylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 % Wasser oder wässrige Phase enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs, enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind z.B. Feuchthaltungsmittel, wie Polyalkohole, z.B. Glycerin, Propylenglykol, Sorbit und/oder Polyethylenglykol, wie Konservierungsmittel, Riechstoffe, etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fette, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasserstoffaufnahmefähigkeit steigernden Fettalkohole, Emulgatoren und/oder Zusätze.

Pasten sind Crémes und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässrig-ethanolische Grundlage auf, der u.a. Polyalkohole, z.B. Glycerin, Glykole, und/oder Polyethylenglykol, als Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Ethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die Erfindung betrifft insbesondere pharmazeutische Präparate in Dosiseinheitsform zur Prophylaxe oder Therapie einer Virusinfektion des Menschen, die als alleinigen Wirkstoff 0,1 mg bis 5 mg einer Verbindung der Formel I zusammen mit einem pharmazeutischen Trägermaterial enthalten.

Die nachfolgenden Beispiele illustrieren die Erfindung, ohne sie in irgendeiner Form einzuschränken.

Beispiel 1: Gruppen von 30 weiblichen Tif:MF2f (SPF)-Mäusen oder weiblichen BALB/c AnCbif Tif (SPF) Mäusen mit einem Körpergewicht von 14-16 g werden unter leichter Narkose mit einem Gemisch aus gleichen Teilen Diethylether, Ethanol und Chloroform mit letalen Dosen (ungefähr eine $LD_{80-90}$; 1-4 plaque forming units [PFU]) in Form von je 0,05 ml einer Suspension von Influenza A/Texas/1/77 (Maus-adaptierter Stamm) Viren intranasal infiziert.

10 von diesen Mäusen werden zum unten angegebenen Zeitpunkt [Tage] bezogen auf den Tag der Infektion einmal (Einzeldosis) die in Tabelle 4 genannten Mengen der jeweiligen Wirksubstanz in 0,05 bzw. in 0,2 ml einer 0,005 Gew.%igen Lösung von Carboxymethylcellulose-natriumsalz in doppelt destilliertem pyrogenfreiem Wasser, im Falle von intranasaler bzw. oraler Applikation auf die in Tabelle 4 genannte Art appliziert.

Die restlichen der obengenannten Mäuse, d.h. 20 dienen zur Kontrolle, d.h. sie erhalten ein Placebo (0,005 Gew.%ige Lösung von Carboxymethylcellulose-natriumsalz).

Die intranasale Applikation der Wirksubstanz wird unter leichter Narkose mit einem Gemisch aus gleichen Teilen Diethylether, Ethanol und Chloroform durchgeführt.

Verbindung I = 2-(1,2-Dipalmitoyl-sn-glycero-3-hydroxyphosphoryloxy)-ethylamin; Verbindung II = 1-Carboxy-2-(1,2-dioleoyl-sn-glycero-3-hydroxyphosphoryloxy)-ethylamin-natriumsalz;
Verbindung III = 2-(3-Palmitoyl-rac-glycero-1-hydroxyphosphoryloxy)-ethylamin;
Verbindung IV = 2-(1-O-Palmitoyl-propandiol-3-O-hydroxyphosphoryloxy)-ethylamin;
Verbindung V = 2-(1,3-Dipalmitoyl-glycero-2-hydroxyphosphoryloxy)-ethylamin.

Tabelle 4

| Wirksubstanz | Applikationsart | Applikationszeit [Tage] | Prozentsatz der 23 Tage nach der Infektion noch lebenden Mäuse in Abhängigkeit von der Wirksubstanzmenge [mg/kg] statistische Signifikanz * P ≤ 0,05 ** P ≤ 0,01(Vierfelder-Test | | | | | |
|---|---|---|---|---|---|---|---|---|
| | | | 10 | 1 | 0,1 | 0,01 | 0,001 | 0 =Kontrolle |
| I | oral | +7 | | 90** | 90** | 90** | | 30 |
| | oral | +7 | 90** | 90** | 100** | | | 30 |
| | intranasal | -7 | | 80** | 90** | 80* | | 20 |
| | intranasal | -7 | | | 50** | 70** | 70** | 5 |
| II | oral | +7 | | 100** | 80* | 100** | | 30 |
| | intranasal | -7 | | 40 | 50 | 80** | | 20 |
| III | intranasal | -7 | | | 60** | 70** | 50* | 5 |
| IV | oral | +7 | | | 10 | 30 | 40* | 0 |
| V | oral | +7 | | | | 80* | 40 | 25 |

<u>Beispiel 2:</u> 4,5 g N-Boc-2-(1,3-dilauroyl-glycero-2-hydroxyphosphoryloxy)-ethylamin-lithiumsalz werden in 50 ml Methylenchlorid gelöst. Die Lösung wird bei 0-5° mit 10 ml Trifluoressigsäure versetzt, 2 Stunden bei 10° ausgerührt und schliesslich unter Vakuum vollständig eingedampft. Der Rückstand wird in ca. 200 ml eines Gemisches von $CHCl_3:CH_3OH:H_2O$ = 1:1,2:1 aufgenommen. Die wässrige Phase wird bei 0-5° mit ca. 10 ml 2 N Natriumhydroxid-Lösung neutralisiert (pH 5,0-5,5). Die organische (untere) Phase wird abgetrennt, auf ca. 1/2 des Volumens eingeengt und bei einer Badtemperatur von 50° mit 60 ml Aceton versetzt. Beim Abkühlen fällt 2-(1,3-Dilauroyl-glycero-2-hydroxyphosphoryloxy)-ethylamin in gut filtrierbarer Form aus; Smp. 144-149°, $R_f$ = 0,51 ($CHCl_3:CH_3OH:H_2O$:Essigsäure = 15:5:1:0,1).

Das Ausgangsmaterial erhält man folgendermassen:

Stufe 2.1: Bei -10° wird eine Lösung von 1,51 ml Phosphoroxychlorid und 3,1 ml Triethylamin in 25 ml Methylenchlorid tropfenweise mit einer Lösung von 6,85 g 1,3-Dilauroyl-glycerin in 50 ml Methylenchlorid versetzt. Das Reaktionsgemisch wird 2 Stunden bei 10° ausgerührt, dann wieder bis 0° abgekühlt und bei dieser Temperatur tropfenweise mit einer Lösung von 2,5 ml Triethylamin und 2,9 g N-Boc-ethanolamin in 10 ml Methylenchlorid versetzt. Das Reaktionsgemisch wird während 1 Stunde bei 30° ausgerührt und anschliessend mit Wasser, Citronensäure- und Natriumhydrogencarbonat-Lösung extrahiert. Die organische Phase wird eingedampft und der ölige Rückstand mit Methylenchlorid-Essigsäureethylester (5:1) an Kieselgel chromatographiert. Aus der Fraktion mit $R_f$ = 0,40 erhält man N-Boc-2-(1,3-dilauroyl-glycero-2-methoxyphosporyloxy)-ethylamin als farbloses Oel.

Stufe 2.2: 4,5 g N-Boc-2-(1,3-dilauroyl-glycero-2-methoxyphosphoryloxy)-ethylamin werden in 45 ml Aceton gelöst. Die Lösung wird mit 1,6 g Lithiumbromid versetzt und während 2 Stunden zum Rückfluss erwärmt. Das Lösungsmittel wird dann unter Vakuum abdestilliert und

der Rückstand in 90 ml eines Gemisches von Chloroform-Methanol-Wasser (1:1,2:1) aufgenommen. Es bilden sich zwei klare Phasen. Die organische (untere) Phase wird eingedampft und am Hochvakuum entgast. Man erhält N-Boc-2-(1,3-dilauroyl-glycero-2-hydroxyphosphoryloxy)-ethylamin-lithiumsalz als Oel, das beim Stehen fest wird; $R_f$ = 0,41 ($CHCl_3$:$CH_3OH$:$NH_3$ [konz.] = 100:20:1).

Beispiel 3: Analog Beispiel 2 erhält man ausgehend von Caprinsäure-2-hydroxyethylester anstelle von 1,3-Dilauroyl-glycerin (vgl. Stufe 2.1) 2-Caprinoyloxyethyl-2-aminoethyl-phosphat als weisses Pulver; Smp. 181-185°, $R_f$ = 0,17 ($CHCl_3$:$CH_3OH$:$H_2O$:Essigsäure = 15:5:1:0,1).

Patentansprüche

1. Verwendung von Kephalinderivaten der Formel I,

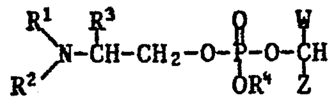

(I)

worin $R^1$ und $R^2$ unabhängig voneinander Wasserstoff oder Niederalkyl, $R^3$ Wasserstoff, Niederalkoxycarbonyl, Carbamoyl oder freies oder geschütztes Carboxy, $R^4$ Wasserstoff oder einen aliphatischen, aromatischen, aromatisch-aliphatischen oder cycloaliphatischen Rest, W Wasserstoff und Z eine 1,2-Dihydroxy-ethyl-, 2-Hydroxy-ethyl-, oder Hydroxymethyl-Gruppe, in der mindestens eine Hydroxygruppe mit einer aliphatischen $C_{6-30}$-Carbonsäure verestert oder mit einem aliphatischen $C_{6-30}$-Alkohol verethert ist, und in der die andere Hydroxygruppe, falls vorhanden, frei, mit einer aliphatischen $C_{2-30}$-Carbonsäure verestert oder mit einem aliphatischen $C_{1-30}$-Alkohol verethert ist, oder W Hydroxymethyl oder eine Hydroxymethylgruppe, die mit einer aliphatischen $C_{6-30}$-Carbonsäure verestert oder mit einem aliphatischen $C_{6-30}$-Alkohol verethert ist und Z eine Hydroxymethylgruppe, die mit einer aliphatischen $C_{6-30}$-Carbonsäure verestert oder mit einem aliphatischen $C_{6-30}$-Alkohol verethert ist, bedeuten, und von pharmazeutisch verwendbaren Salzen dieser Verbindungen zur Herstellung von pharmazeutischen Präparaten für die Anwendung zur Prophylaxe und Therapie von Virusinfektionen bei Warmblütern einschliesslich des Menschen.

2. Verwendung nach Anspruch 1 von Verbindungen der Formel I, worin $R^1$ und $R^2$ unabhängig voneinander Wasserstoff oder $C_{1-2}$-Alkyl, $R^3$ Wasserstoff, Carboxy oder Benzyloxycarbonyl, $R^4$ Wasserstoff, Benzyl, 2-Amino-ethyl, 2,3-Dihydroxy-propyl, Trimethylsilyl, 2,3,4,5,6-Pentahydroxy-cyclohexyl oder 2-Hydroxy-3-glycyloxy-propyl, W Wasserstoff und Z eine 1,2-Dihydroxyethyl- oder 2-Hydroxyethylgrupe, in der jeweils die 2-Hydroxygruppe mit einer geradkettigen Alkansäure mit 6, 10, 12, 14, 16, 18 oder 20 C-Atomen oder mit

einer geradkettigen $C_{18}$-Alkensäure mit 1-3 Doppelbindungen verestert oder mit einem geradkettigen Alkanol mit 6, 8, 16 oder 18 C-Atomen verethert ist und in der die 1-Hydroxygruppe, falls vorhanden, entweder frei oder mit einer geradkettigen Alkansäure mit 6, 10, 12, 14, 16, 18 oder 20 C-Atomen oder mit einer geradkettigen $C_{18}$-Alkensäure mit 1-2 Doppelbindungen verestert oder mit einem geradkettigen Alkanol mit 1, 6, 8, 16 oder 18 C-Atomen verethert ist, oder W Hydroxymethyl oder Palmitoyloxymethyl und Z Palmitoyloxymethyl bedeuten, und von pharmazeutisch verwendbaren Salzen dieser Verbindungen.

3. Verwendung nach Anspruch 1 von Verbindungen der Formel I, worin $R^1$ und $R^2$ Wasserstoff, $R^3$ Wasserstoff oder Carboxy, $R^4$ Wasserstoff, W Wasserstoff und Z eine 1,2-Dihydroxy-ethylgruppe, in der die 2-Hydroxygruppe mit einer geradkettigen $C_{14-18}$-Alkansäure mit gerader Anzahl von C-Atomen oder mit einer geradkettigen, einfach ungesättigten $C_{18}$-Alkensäure verestert oder mit einem geradkettigen Alkanol mit 16 oder 18 C-Atomen verethert ist und in der die 1-Hydroxygruppe entweder frei oder mit einer geradkettigen $C_{14-18}$--Alkansäure mit gerader Anzahl von C-Atomen oder mit einer geradkettigen, einfach ungesättigten $C_{18}$-Alkensäure verestert oder mit einem geradkettigen Alkanol mit 1, 16 oder 18 C-Atomen verethert ist, bedeuten, und ihrer pharmazeutisch verwendbaren Salze.

4. Verwendung nach einem der Ansprüche 1 - 3 von Verbindungen der Formel I, worin W Wasserstoff und Z eine 1,2-Dihydroxy-ethylgruppe, in der die 2-Hydroxygruppe mit einer geradkettigen $C_{14-18}$-Alkansäure mit gerader Anzahl von C-Atomen oder mit einer geradkettigen, einfach ungesättigten $C_{18}$-Alkensäure verestert ist und in der die 1-Hydroxygruppe entweder frei oder mit einer geradkettigen $C_{14-18}$-Alkansäure mit gerader Anzahl von C-Atomen oder mit einer geradkettigen, einfach ungesättigten $C_{18}$-Alkensäure verestert ist, bedeuten, und ihrer pharmazeutisch verwendbaren Salze.

5. Verwendung nach einem der Ansprüche 1-3 von Verbindungen der Formel I, worin W Wasserstoff und Z eine 1,2-Dihydroxyethylgruppe, in der die beiden Hydroxygruppen mit einer geradkettigen $C_{14-18}$-Alkansäure mit gerader Anzahl von C-Atomen oder mit einer geradkettigen, einfach ungesättigten $C_{18}$-Alkensäure verestert sind, oder W und Z je eine Hydroxymethylgruppe, die mit einer geradkettigen $C_{14-18}$-Alkansäure mit gerader Anzahl von C-Atomen oder mit einer geradkettigen, einfach ungesättigten $C_{18}$-Alkensäure verestert ist, bedeuten, und ihrer pharmazeutisch verwendbaren Salze.

6. Verwendung nach Anspruch 1 von 2-(1,2-Dipalmitoyl-sn-glycero-3-hydroxyphosphoryloxy)-ethylamin.

7. Verwendung einer Verbindung der Formel I oder eines pharmazeutisch verwendbaren Salzes davon nach einem der Ansprüche 1 - 6 zur Herstellung von pharmazeutischen Präparaten für die Anwendung zur Prophylaxe oder Therapie von Infektionen durch Paramyxoviridae, Orthomyxoviridae, Picornaviridae, Chordopoxvirinae oder Alphaherpesvirinae.

8. Verwendung einer Verbindung der Formel I oder eines pharmazeutisch verwendbaren Salzes davon nach einem der Ansprüche 1 - 6 zur Herstellung von pharmazeutischen Präparaten für die Anwendung zur Prophylaxe oder Therapie von Infektionen durch Parainfluenza, Herpes simplex, Encephalomyocarditis oder Vaccinia Viren.

9. Verwendung einer Verbindung der Formel I oder eines pharmazeutisch verwendbaren Salzes davon nach einem der Ansprüche 1 - 6 zur Herstellung von pharmazeutischen Präparaten für die Anwendung zur Prophylaxe oder Therapie von Infektionen durch Influenza Viren.

10. Kephalinderivate der Formel I,

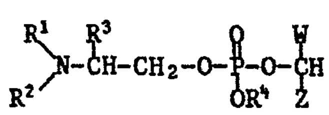

(I)

worin $R^1$ und $R^2$ unabhängig voneinander Wasserstoff oder Niederalkyl, $R^3$ Wasserstoff, Niederalkoxycarbonyl, Carbamoyl oder freies oder geschütztes Carboxy, $R^4$ Wasserstoff oder einen aliphatischen, aromatischen, aromatisch-aliphatischen oder cycloaliphatischen Rest, W Wasserstoff und Z eine 1,2-Dihydroxy-ethyl-, 2-Hydroxy-ethyl-, oder Hydroxymethyl-Gruppe, in der mindestens eine Hydroxygruppe mit einer aliphatischen $C_{6-30}$-Carbonsäure verestert oder mit einem aliphatischen $C_{6-30}$-Alkohol verethert ist, und in der die andere Hydroxygruppe, falls vorhanden, frei, mit einer aliphatischen $C_{2-30}$-Carbonsäure verestert oder mit einem aliphatischen $C_{1-30}$-Alkohol verethert ist, oder W Hydroxymethyl oder eine Hydroxymethylgruppe, die mit einer aliphatischen $C_{6-30}$-Carbonsäure verestert oder mit einem aliphatischen $C_{6-30}$-Alkohol verethert ist, und Z eine Hydroxymethylgruppe, die mit einer aliphatischen $C_{6-30}$-Carbonsäure verestert oder mit einem aliphatischen $C_{6-30}$-Alkohol verethert ist, bedeuten, wobei $R^1$ von Wasserstoff und $C_{1-2}$-Alkyl, oder $R^2$ von Wasserstoff und $C_{1-2}$-Alkyl, oder $R^3$ von Wasserstoff, Carboxy und Benzyloxycarbonyl, oder $R^4$ von Wasserstoff, Benzyl, 2-Amino-ethyl, 2,3-Dihydroxypropyl, Trimethylsilyl, 2,3,4,5,6-Pentahydroxy-cyclohexyl und 2-Hydroxy-3-glycyloxy-propyl, oder, falls W für Wasserstoff steht, Z von einer 1,2-Dihydroxyethyl- und 2-Hydroxy-ethylgruppe, in der jeweils die 2-Hydroxygruppe mit einer geradkettigen Alkansäure mit 6, 10, 12, 14, 16, 18 oder 20 C-Atomen oder mit einer geradkettigen $C_{18}$-Alkensäure mit 1-3 Doppelbindungen verestert oder mit einem geradkettigen Alkanol mit 6, 8, 16 oder 18 C-Atomen verethert ist und in der die 1-Hydroxygruppe, falls vorhanden, entweder frei oder mit einer geradkettigen Alkansäure mit 6, 10, 12, 14, 16, 18 oder 20 C-Atomen oder mit einer geradkettigen $C_{18}$-Alkensäure mit 1-2 Doppelbindungen verestert oder mit einem geradkettigen Alkanol mit 1, 6, 8, 16 oder 18 C-Atomen verethert ist, oder, falls W nicht für Wasserstoff steht, W von Palmitoyloxymethyl und Hydroxymethyl, oder, falls W nicht für Wasserstoff steht, Z von Palmitoyloxymethyl verschieden ist, und Salze dieser Verbindungen.

11. Verbindungen der Formel I nach Anspruch 10, worin $R^1$ und $R^2$ unabhängig voneinander Wasserstoff oder Niederalkyl, $R^3$ Wasserstoff, Carboxy oder Niederalkoxycarbonyl, $R^4$ Wasserstoff und W und Z je mit einer geradkettigen $C_{6-22}$-Alkansäure, die von Palmitinsäure verschieden ist, oder mit einer geradkettigen $C_{18}$-Alkensäure mit 1-3 Doppelbindungen verestertes Hydroxymethyl bedeuten, und ihre pharmazeutisch verwendbaren Salze.

12. 2-(1,3-Dilauroyl-glycero-2-hydroxyphosphoryloxy)-ethylamin nach Anspruch 10.

13. Eine Verbindung nach Anspruch 10, 11 oder 12 zur Anwendung in einem Verfahren zur therapeutischen Behandlung des menschlichen oder tierischen Körpers.

14. Pharmazeutische Präparate in Dosiseinheitsform zur Prophylaxe oder Therapie einer Virusinfektion des Menschen, die als alleinigen Wirkstoff 0,1 mg bis 5 mg einer Verbindung der Formel I gemäss einem der Ansprüche 1-6 und 10-12 zusammen mit einem pharmazeutischen Trägermaterial enthalten.

15. Pharmazeutische Präparate, die als Wirkstoff eine Verbindung nach Anspruch 10, 11 oder 12 zusammen mit einem pharmazeutischen Trägermaterial enthalten.

16. Pharmazeutische Präparate, die als alleinigen Wirkstoff eine Verbindung nach Anspruch 11 oder 12 zusammen mit einem pharmazeutischen Trägermaterial enthalten.

FO 7.4 VBU/bg*/sm*